# EUROPEAN PATENT SPECIFICATION

(11) **EP 2 515 896 B1**
(45) Date of publication and mention of the grant of the patent: **31.07.2019**
(21) Application number: 10803388.7
(22) Date of filing: 22.12.2010
(51) Int. Cl.: A61K 31/192, A61K 31/21, A61P 7/02

(54) **ANTICOAGULANT COMPOUNDS AND THEIR USE**
ANTIKOAGULANSVERBINDUNGEN UND IHRE VERWENDUNG
COMPOSÉS ANTICOAGULANTS ET UTILISATION DE CES DERNIERS

(30) Priority: 23.12.2009 GB 0922510
(43) Date of publication of application: 31.10.2012
(73) Proprietor: Haomamedica Limited, Colchester, Essex CO7 9DS (GB)
(72) Inventor: HODGES, Stephen, Essex CO7 9DS (GB); SOPER, Robin, Essex CO7 9DS (GB)
(74) Representative: CMS Cameron McKenna Nabarro Olswang LLP
(86) International application number: PCT/GB2010/052194
(87) International publication number: WO 2011/077158

(56) References cited:
- US-A- 4 734 282
- US-A- 5 180 742
- RONDEN JACINTHA E ET AL: "Natural prenylquinones inhibit the enzymes of the vitamin K cycle in vitro", BIOCHIMICA ET BIOPHYSICA ACTA, vol. 1298, no. 1, 1996, pages 87-94, XP002632746, ISSN: 0006-3002
- NAGANATHAN SRIRAM ET AL: "The active site of vitamin K and the role of the vitamin K-dependent carboxylase", JOURNAL OF THE AMERICAN CHEMICAL SOCIETY, vol. 116, no. 22, 1994, pages 9831-9839, XP002632747, ISSN: 0002-7863
- DHAON M K ET AL: "DERIVATIVES OF 2 METHYL-1 4-NAPHTHOQUINONE AS SUBSTRATES AND INHIBITORS OF THE VITAMIN K-DEPENDENT CARBOXYLASE", JOURNAL OF MEDICINAL CHEMISTRY, vol. 27, no. 9, 1984, pages 1196-1201, XP002632748, ISSN: 0022-2623
- RISHAVY M A ET AL: "Bronsted analysis reveals Lys218 as the carboxylase active site base that deprotonates vitamin K hydroquinone to initiate vitamin K-dependent protein carboxylation", BIOCHEMISTRY 20061107 AMERICAN CHEMICAL SOCIETY US, vol. 45, no. 44, 7 November 2006 (2006-11-07), pages 13239-13248, XP002632749, DOI: DOI:10.1021/BI0609523

## Description

The present invention relates to anticoagulant compounds and uses thereof.

Anticoagulants are given to stop inappropriate blood clotting, and disorders resulting therefrom, and may be used inter alia in prevention of deep vein thrombosis, pulmonary embolism, myocardial infarctions and strokes, maintaining mechanical heart valve function following mechanical, xeno-, homo- or autologous heart valve replacement and prevention of clot formation during surgery.

Warfarin and heparin are commonly used anticoagulants with bleeding being the most common complication of their therapeutic use.

It has now surprisingly been found that certain naphthoquinone compounds are able to act as anticoagulants. Thus, the present invention relates to an alternative anticoagulant.

The invention is as claimed in claims 1-14
In an aspect of the present invention, the compound of formula (I) is not vitamin K₃.

Disclosed herein are pharmaceutical compositions comprising a compound of formula (I) with a pharmaceutically acceptable carrier. In a further aspect the invention relates to a pharmaceutical composition comprising a compound of formula (I) and a pharmaceutically acceptable carrier, diluent or excipient therefor, for use as an anticoagulant. The disclosure also relates to use of a compound of formula I, or pharmaceutical composition comprising a compound of formula I, in the manufacture of a medicament for use as an anticoagulant.

The term "composition", as in pharmaceutical composition, is intended to encompass a product comprising the active ingredient(s), and the inert ingredient(s) (pharmaceutically acceptable excipients) that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing a compound of Formula I, additional active ingredient(s), and pharmaceutically acceptable excipients. Suitable pharmaceutical compositons may be found in, for example, Remington The Science and Practice of Pharmacy, 19th ed., Mack Printing Company, Easton, Pennsylvania (1995). For parenteral administration, a parenterally acceptable aqueous solution may be employed, which is pyrogen free and has requisite pH, isotonicity, and stability. Suitable solutions will be well known to the skilled person, with numerous methods being described in the literature. A brief review of methods of drug delivery may also be found in e.g. Langer, Science (1990) 249, 1527.

Reference to the compound of formula (I) herein is taken to include reference to all pharmaceutically acceptable solvates or, more particularly, salts, or tautomers, unless otherwise apparent from the context. Accordingly in its broadest aspect the present invention relates to compounds of formula (I) or a pharmaceutically acceptable solvate or, more particularly, salt, or tautomer thereof, for use as anticoagulants.

The term "pharmaceutically acceptable salts" refers to salts prepared from pharmaceutically acceptable non-toxic bases including inorganic bases and organic bases (for example, given the presence of substituent -CO₂R^{a} present in R³), or salts prepared from pharmaceutically acceptable non-toxic acids including inorganic acids and organic acids (for example, in the case where a basic substituent is present in any of R¹ or R²).

Salts derived from inorganic bases include aluminum, ammonium, calcium, copper, ferric, ferrous, lithium, magnesium, potassium, sodium, zinc, and the like. Salts derived from pharmaceutically acceptable organic non-toxic bases include salts of primary, secondary, and tertiary amines, substituted amines including naturally occurring substituted amines, cyclic amines, and basic ion exchange resins, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, isopropylamine, lysine, morpholine, piperazine, piperidine, polyamine resins, procaine, purines, theobromine, triethylamine, trimethylamine, tripropylamine, and the like.

Salts derived from acids include acetic, benzenesulfonic, benzoic, camphorsulfonic, citric, ethanesulfonic, fumaric, glutamic, hydrobromic, hydrochloric, isethionic, lactic, maleic, mandelic, methanesulfonic, nitric, pamoic, pantothenic, phosphoric, succinic, sulfuric, tartaric, p-toluenesulfonic acid, and the like.

As mentioned above, also encompassed by formula I are any solvates of the compounds and their salts. Preferred solvates are solvates formed by the incorporation into the solid state structure (e.g. crystal structure) of the compounds of the invention of molecules of a non-toxic pharmaceutically acceptable solvent (referred to below as the solvating solvent). Examples of such solvents include water, alcohols (such as ethanol, isopropanol and butanol) and dimethylsulphoxide. Solvates can be prepared by recrystallising the compounds of the invention with a solvent or mixture of solvents containing the solvating solvent. Whether or not a solvate has been formed in any given instance can be determined by subjecting crystals of the compound to analysis using well known and standard techniques such as thermogravimetric analysis (TGE), differential scanning calorimetry (DSC) and X-ray crystallography.

The solvates can be stoichiometric or non-stoichiometric solvates. Particularly preferred solvates are hydrates, and examples of hydrates include hemihydrates, monohydrates and dihydrates.

For a more detailed discussion of solvates and the methods used to make and characterise them, see Bryn et al., Solid-State Chemistry of Drugs, Second Edition, published by SSCI, Inc of West Lafayette, IN, USA, 1999, ISBN 0-967-06710-3.

The present invention also includes within its scope the use of prodrugs of the compounds of formula (I). In general, such prodrugs are functional derivatives of the compounds of formula (I) which are readily convertible *in vivo* into the required compound. Conventional procedures for the selection and preparation of suitable prodrug derivatives are well known in the art.

The term "prodrug" of a relevant compound of formula I includes any compound that, following administration (e.g. oral or parenteral administration), is metabolised *in vivo* to form that compound in an experimentally-detectable amount, and within a predetermined time (e.g. within a dosing interval of between 6 and 24 hours (i.e. once to four times daily)).

Prodrugs of compounds of formula I may be prepared by modifying functional groups present on the compound in such a way that the modifications are cleaved, *in vivo* when such prodrug is administered to a mammalian subject. The modifications typically are achieved by synthesizing the parent compound with a prodrug substituent. Prodrugs include compounds of formula I wherein a hydroxyl, amino, sulfhydryl, carboxyl or carbonyl group in a compound of formula I is bonded to any group that may be cleaved *in vivo* to regenerate the free hydroxyl, amino, sulfhydryl, carboxyl or carbonyl group, respectively.

Examples of prodrugs include, but are not limited to, esters and carbamates of hydroxyl functional groups, esters groups of carboxyl functional groups, N-acyl derivatives and N-Mannich bases. General information on prodrugs may be found e.g. in Bundegaard, H. "Design of Prodrugs" p. I-92, Elsevier, New York-Oxford (1985).

In one aspect the prodrug is not vitamin K. Unless otherwise stated, the term "vitamin K" as used herein relates to vitamin K₁ and vitamin K₂ collectively and not to man-made analogues of vitamin K.

Compounds of formula I contain double bonds and may thus exist as *E* (*entgegen*) and *Z* (*zusammen*) geometric isomers about each individual double bond. All such isomers and mixtures thereof are included within the scope of the invention.

Compounds of formula I may exist as regioisomers and may also exhibit tautomerism. All tautomeric forms and mixtures thereof are included within the scope of the invention.

Compounds of formula I may contain one or more asymmetric carbon atoms and may therefore exhibit optical and/or diastereoisomerism. Diastereoisomers may be separated using conventional techniques, e.g. chromatography or fractional crystallisation. The various stereoisomers may be isolated by separation of a racemic or other mixture of the compounds using conventional, e.g. fractional crystallisation or HPLC, techniques. Alternatively the desired optical isomers may be made by reaction of the appropriate optically active starting
materials under conditions which will not cause racemisation or epimerisation (i.e. a 'chiral pool' method), by reaction of the appropriate starting material with a 'chiral auxiliary' which can subsequently be removed at a suitable stage, by derivatisation (i.e. a resolution, including a dynamic resolution), for example with a homochiral acid followed by separation of the diastereomeric derivatives by conventional means such as chromatography, or by reaction with an appropriate chiral reagent or chiral catalyst all under conditions known to the skilled person. All stereoisomers and mixtures thereof are included within the scope of the invention.

The compound of formula (I), or pharmaceutical compositions comprising the compound of formula (I), for use mentioned in the above-mentioned aspects of the invention may be utilised in a method of medical treatment. Thus, according to further aspects of the disclosure, there is provided:
(i) the use of a compound formula (I), or pharmaceutical composition comprising a compound of formula (I), for the manufacture of a medicament for use as an anticoagulant; and
(ii) a method of treatment or prevention of a disorder or condition benefitting from anticoagulant therapy, which method comprises the administration of an effective amount of a compound of formula (I), or a pharmaceutical composition comprising the compound of formula (I), to a patient in need of such treatment.

The term "disorder or condition benefitting from anticoagulant therapy" will be understood by those skilled in the art to include: thrombosis, and diseases related thereto. For example diseases related to thrombosis include deep vein thrombosis (particularly prevention of deep vein thrombosis), pulmonary embolism, myocardial infarction (e.g. myocardial infarction in surgical procedures requiring anticoagulation), strokes, and maintaining heart valve function for mechanical or transplanted hearts, such as human or, particularly, non-human hearts (e.g. following mechanical, xeno-, homo- or autologous heart valve replacement and prevention of clot formation during surgery).

Thus, in one aspect the compound of formula (I) is used as an anticoagulant to prevent thrombosis and diseases related to thrombosis. Particular disorders or conditions that may be mentioned in relation to the aspects of the invention described hereinbefore include thrombosis.

The compounds and compositions of the invention having anticoagulant activity may be used for the prevention or treatment of thrombosis, and diseases related thereto, for example in prevention of deep vein thrombosis, pulmonary embolism, myocardial infarctions in surgical procedures requiring anticoagulation, strokes, and maintaining heart valve function for mechanical or transplanted hearts, such as human hearts or, particularly as non human hearts (e.g. following mechanical, xeno-, homo- or autologous heart valve replacement and prevention of clot formation during surgery).

Thus, further aspects of the disclosure relate to the following.
(a) A compound of formula I, or pharmaceutical composition comprising a compound of formula (I), as hereinbefore defined, for use in the treatment or prevention of a condition or disorder selected from thrombosis, deep vein thrombosis, pulmonary embolism, myocardial infarction in surgical procedures requiring anticoagulation, strokes, and maintaining heart valve function for mechanical or transplanted hearts (e.g. thrombosis).
(b) Use of a compound of formula I, or pharmaceutical composition comprising a compound of formula (I), as hereinbefore defined, for the preparation of a medicament for the treatment or prevention of a condition or disorder selected from thrombosis, deep vein thrombosis, pulmonary embolism, myocardial infarction in surgical procedures requiring anticoagulation, strokes, and maintaining heart valve function for mechanical or transplanted hearts (e.g. thrombosis).
(c) A method of treatment or prevention of a disorder or condition selected from thrombosis, deep vein thrombosis, pulmonary embolism, myocardial infarction in surgical procedures requiring anticoagulation, strokes, and maintaining heart valve function for mechanical or transplanted hearts (e.g. thrombosis), which method comprises the administration of an effective amount of a compound of formula (I), or a pharmaceutical composition comprising the compound of formula (I), to a patient in need of such treatment.

For the avoidance of doubt, in the context of the present invention, the term *"treatment"* includes references to therapeutic or palliative treatment of patients in need of such treatment, as well as to the prophylactic treatment and/or diagnosis of patients which are susceptible to the relevant disease states.

The terms *"patient"* and *"patients"* include references to mammalian (e.g. human) patients.

The term "effective amount" refers to an amount of a compound, which confers a therapeutic effect on the treated patient (e.g. sufficient to treat or prevent the disease). The effect may be objective (i.e. measurable by some test or marker) or subjective (i.e. the subject gives an indication of or feels an effect).

The term "halogen" as used herein includes fluorine, chlorine, bromine and iodine (e.g. bromine or, more particularly, chlorine of fluorine).

The term "hydrocarbon" as used herein with reference to any of R¹, R², R³, R^{a} and R^{b} includes alkyl, alkenyl, alkynyl, cycloalkyl, alkyl, aryl, aryl-alkyl, aryl-alkenyl and aryl-alkynyl.

Suitable alkyl groups include straight chained or branched alkyl groups containing from 1 to 18 carbon atoms, or more preferably 1 to 9 carbon atoms. For example, typical examples can include methyl or ethyl, or straight chained or branched propyl, butyl, pentyl, hexyl, heptyl, octyl, nonyl or the like.

Suitable alkenyl groups include straight chained and branched alkenyl groups containing from 2 to 18 carbon atoms, and may include vinyl, allyl or isoprene moieties, 2-, 3- or 4- pentenyl, or 2-, 3-, or 4- hexenyl or the like, and isomeric forms thereof. Alkenyl groups as present in the compounds of the invention may include one or more degrees of unsaturation.

Suitable alkynyl groups include straight chained and branched alkynyl groups containing from 2 to 18 carbon atoms. For example, typical examples can include ethynyl and propynyl groups.

Suitable cycloalkyl groups include groups containing from 3 to 7 carbon atoms, for example cyclopropyl or cyclohexyl.

Suitable aryl groups can include aromatic hydrocarbon systems having one ring or two or three fused rings, such as phenyl or naphthyl. A particularly suitable aryl group can be phenyl.

Suitable heterocyclic groups can include ring systems having 5 or 6 ring atoms of which at least one ring atom is oxygen, sulphur or nitrogen. The ring systems can be aromatic or non-aromatic. Examples can include piperazinyl, morpholinyl, pyrrolyl, imidazolyl, thienyl, furanyl or other known heterocyclic ring systems.

According to a preferred embodiment of the present invention, R¹ represents a hydrocarbon group comprising a straight chain, branched or cyclic group each containing up to 18 carbon atoms. More preferably R¹ represents an alkyl group including straight chained or branched alkyl groups containing from 1 to 18 carbon atoms, or more preferably 1 to 9 carbon atoms (e.g. 1 to 6 carbon atoms, such as 1 to 5 carbon atoms). It is particularly preferred that R¹ represents methyl.

According to a further preferred embodiment of the present invention, n represents 0 or, alternatively, n is 4 and R², at each occurrence, is hydrogen (e.g. R² represents hydrogen and n is 4).

Disclosed herein, R³ represents a hydrocarbon group comprising a straight chained or branched hydrocarbon group containing up to 18 carbon atoms, preferably an appropriate alkyl or alkenyl group, substituted by at least one moiety including a -CO₂R^{a} substituent. More preferably, R³ represents C₁₋₉ alkyl or C₂₋₉ alkenyl, which can be straight chained or branched, substituted by at least one moiety including a -CO₂R^{a} substituent, wherein R^{a} is substantially as hereinbefore defined. Preferably in the context of R³, R^{a} represents hydrogen, or a hydrocarbon group comprising a straight chained or branched hydrocarbon group containing up to 18 carbon atoms, preferably up to 9 carbon atoms, and even more preferably up to 6 carbon atoms. Preferably R^{a} represents hydrogen or C₁₋₆ straight chained or branched alkyl, in particular methyl.

Preferably, disclosed herein, R³ can be represented by the following formula (II) 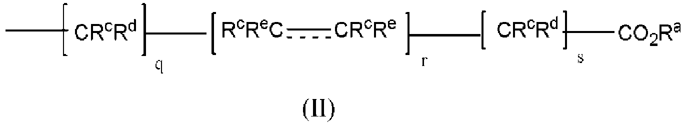 where the unattached bond represents the point of attachment of the structural fragment of formula (II) to the rest of the compound of Formula (I);
R^{a} is as hereinbefore defined;
R^{c}, R^{d} and R^{e} are independently selected from hydrogen or C₁₋₆ alkyl (which can be straight chained or branched);
q is 1, 2, 3 or 4;
r and s are independently selected from 0, 1, 2, 3 or 4;
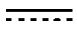 represents a single or double bond, and when this is a double bond R^{e} is not present in above formula (II).

Preferably formula (II) represents a C₄₋₈ straight chained or branched alkyl group, or a C₄₋₈ straight chained or branched alkenyl group, substituted by -CO₂R^{a}, wherein preferably R^{a} represents hydrogen or C₁₋₆ straight chained or branched alkyl, in particular methyl (e.g. R^{a} when attached to R³ represents H or CH₃).

Disclosed herein, especially preferred groups represented by formula (II) include:

-(CH₂)₇CO₂H;

and

-CH₂CH=C(CH₃)CO₂H.

According to the invention, the group represented by formula (II) is -CH₂CH=C(CH₃)(CH₂)₂CO₂H;

Specifically, the present disclosure provides one or more of the following compounds:
(i) 2,3-dimethoxy-1,4-naphthoquinone (XVI);
(ii) menadione (III);
(iii) KCAT-5C-Me (XIX);
(iv) NaQuinate-Me (VII);
(v) (4E)-6-(1,4-dihydro-2-methyl-1,4-dioxonaphthalen-3-yl)-4-methylhex-4-enoic acid (VIII);
(vi) (2E)-4-(1,4-dihydro-2-methyl-1,4-dioxonaphthalen-3-yl)-2-methylbut-2-enoic acid (XIV); and
(vii) 8-(1,4-dihydro-2-methyl-1,4-dioxonaphthalen-3-yl)octanoic acid (XV),
for use as an anticoagulant.

For the avoidance of doubt, if there is a conflict between the given chemical name and the chemical structure, the chemical structure predominates.

Especially preferred is (4E)-6-(1,4-dihydro-2-methyl-1,4-dioxonaphthalen-3-yl)-4-methylhex-4-enoic acid (VIII) for use in therapy according to the present invention substantially as hereinbefore described.

The present disclosure also provides novel compounds for use in therapy according to the present invention substantially as hereinbefore described. Specifically, these novel compounds are (2E)-4-(1,4-dihydro-2-methyl-1,4-dioxonaphthalen-3-yl)-2-methylbut-2-enoic acid (XIV) and 8-(1,4-dihydro-2-methyl-1,4-dioxonaphthalen-3-yl)octanoic acid (XV).

In a particular embodiment, the compound of formula (I) is not menadione.

The disclosure also relates to use of a compound of formula I, or pharmaceutical composition comprising a compound of formula I, in the manufacture of a medicament for use as an anticoagulant.

The disclosure also relates to use of the compound of formula (I), or pharmaceutical compositions comprising the compound of formula (I), in the preparation of a medicament for prevention of thrombosis, or diseases caused thereby, in a patient who is to undertake a surgical procedure. Thus the invention also relates to a compound of formula (I), or pharmaceutical compositions comprising the compound of formula (I), for use in the prevention of thrombosis, or diseases caused thereby, in a patient who is to undertake a surgical procedure and, disclosed herein, to a method of preventing thrombosis, or diseases caused thereby, in a patient who is to undertake a surgical procedure by administration of an effective amount of the compound of formula (I), or pharmaceutical compositions comprising the compound of formula (I), to the patient (e.g. in advance of surgery or after surgery).

In one aspect the use of the compound of the invention may be as part of a combined therapy along with another therapeutic agent.

The disclosure also relates to a combination of a compound of formula (I) with a coagulant, such as vitamin K. In one aspect the compound of formula (I) is to modulate the coagulation effect of the coagulant (e.g. the compound of formula (I) modulates the coagulation effect of the coagulant). In another aspect, the coagulant is provided to modulate the anticoagulation effect of the compound of formula (I).

The disclosure also relates to use of vitamin K in preparation of a medicament for reversal of the anticoagulant effects of the compound of formula (I).

In another aspect of the disclosure there is provided a method for the treatment or prevention of diseases caused by blood coagulation, which comprises administering to a patient in need of treatment a therapeutically effective amount of a compound or composition of the invention (e.g. a compound of formula (I) or a pharmaceutical composition comprising the compound of formula (I)). Thus, there is also provided a compound of formula (I), or a pharmaceutical composition comprising the compound of formula (I), for use in the treatment or prevention of diseases caused by blood coagulation and the use of a compound of formula (I), or a pharmaceutical composition comprising the compound of formula (I), for the preparation of a medicament for the treatment or prevention of diseases caused by blood coagulation.

In another aspect of the present disclosure there is provided a method for the management of the blood clotting capability of a patient, the method comprising administering to a patient in need of treatment a therapeutically effective amount of a compound or composition of the invention (e.g. a compound of formula (I) or a pharmaceutical composition comprising the compound of formula (I)), to exert an anticoagulant effect, then administration of a coagulant to modulate or reverse the anticoagulant effect. Thus, the invention provides a compound of
formula (I), or a pharmaceutical composition comprising the compound of formula (I), for use in a method for the management of the blood clotting capability of a patient, the method comprising administering to a patient in need of treatment a therapeutically effective amount of a compound or composition of the invention (e.g. a compound of formula (I) or a pharmaceutical composition comprising the compound of formula (I)), to exert an anticoagulant effect, then administration of a coagulant to modulate or reverse the anticoagulant effect and the use of a compound of formula (I), or, disclosed herein, a pharmaceutical composition comprising the compound of formula (I), for the preparation of a medicament for use in a method for the management of the blood clotting capability of a patient, the method comprising administering to a patient in need of treatment a therapeutically effective amount of a compound or composition of the invention (e.g. a compound of formula (I) or a pharmaceutical composition comprising the compound of formula (I)), to exert an anticoagulant effect, then administration of a coagulant to modulate or reverse the anticoagulant effect.

By way of example, a patient who needs to undergo surgery and who is on an anticoagulant needs to be treated with a coagulant to prevent excessive bleeding following surgery. The use of the compound of formula (I), and pharmaceutical compositions comprising the compound of formula (I), as anticoagulants allows for efficient and quick reversal of anticoagulant effect by the use of vitamin K (e.g. vitamin K₁ or K₂) and its rapid elimination from the body. In other words, the use of a compound of formula (I), and pharmaceutical compositions comprising a compound of formula (I), as an anticoagulant enables one to quickly and efficiently reverse the anticoagulant effect by:
(a) adding vitamin K (e.g. vitamin K₁ or K₂); and/or
(b) stopping administration of the compound of formula (I), as the compound of formula (I) is rapidly eliminated from the body.

In contrast, reversal of the effect of warfarin, another anticoagulant, is more time consuming and takes longer to achieve, as warfarin has a relatively long biological half-life (2.5.days).

Thus in one aspect the disclosure relates to a treatment regime comprising treatment of a patient with the compound of formula (I), or pharmaceutical compositions comprising the compound of formula (I), then treatment of the same patient with vitamin K, or other coagulant, following surgery, to allow for blood clotting to occur. Optionally the patient can be treated with the compound of formula (I), or pharmaceutical compositions comprising the compound of formula (I), after the surgery has been completed. The invention relates to use of the compound of formula (I), or pharmaceutical compositions comprising the compound of formula (I), in such a regime, and, disclosed herein, in the preparation of a medicament for use in such a regime.

In one aspect the disclosure relates to a combination of a compound of formula I with another active component, for example another anticoagulant.

The compounds may be used together as a combined preparation for simultaneous, separate or sequential use.

In accordance with the invention, compounds of formula (I) may be administered alone (i.e. as a monotherapy, such as a monotherapy for the prevention or treatment of thrombosis etc). In alternative embodiments of the invention, however, compounds of formula (I) may be administered in combination with another therapeutic agent (e.g. another therapeutic agent for the prevention or treatment of thrombosis or, alternatively, with a coagulant compound (e.g. vitamin K)).

Thus further aspects of the disclosure relate to a combination product comprising:
(A) a compound of formula (I), as hereinbefore defined, and
(B) another therapeutic agent (e.g. a coagulant (e.g. vitamin K) or an anticoagulant),
wherein each of components (A) and (B) is formulated in admixture with a pharmaceutically-acceptable adjuvant, diluent or carrier.

When used herein, the term *"another therapeutic agent"* includes references to one or more (e.g. one) therapeutic agents (e.g. one therapeutic agent) selected from coagulants and/or anticoagulants.

Particular other therapeutic agents that may be mentioned include, for example, the coagulants vitamin K₁ and vitamin K₂ and the anticoagulant, warfarin.

When used herein, the term *"administered sequentially, simultaneously or concomitantly"* includes references to:
administration of separate pharmaceutical formulations (one containing the compound of formula I and one or more others containing the one or more other therapeutic agents); and
administration of a single pharmaceutical formulation containing the compound of formula I and the other therapeutic agent(s).

The combination product described above provides for the administration of component (A) in conjunction with component (B), and may thus be presented either as separate formulations, wherein at least one of those formulations comprises component (A) and at least one comprises component (B), or may be presented (i.e. formulated) as a combined preparation (i.e. presented as a single formulation including component (A) and component (B)).

Thus, there is further provided:
(I) a pharmaceutical formulation including a compound of formula I, as hereinbefore defined and another therapeutic agent, in admixture with a pharmaceutically-acceptable adjuvant, diluent or carrier (which formulation is hereinafter referred to as a "combined preparation"); and
(II) a kit of parts comprising components:
   (i) a pharmaceutical formulation including a compound of formula I, as hereinbefore defined, in admixture with a pharmaceutically-acceptable adjuvant, diluent or carrier; and
   (ii) a pharmaceutical formulation including another therapeutic agent, in admixture with a pharmaceutically-acceptable adjuvant, diluent or carrier,
which components (i) and (ii) are each provided in a form that is suitable for administration in conjunction with the other.

Component (i) of the kit of parts is thus component (A) in admixture with a pharmaceutically-acceptable adjuvant, diluent or carrier. Similarly, component (ii) is component (B) in admixture with a pharmaceutically-acceptable adjuvant, diluent or carrier.

In one aspect the disclosure relates to a method of preparing a combined medicine, the method comprising combining a coagulant, for example vitamin K, with the compound of formula (I).

Optionally the combined medicine may then be combined with any pharmaceutically acceptable excipient, diluent or carrier to form a pharmaceutical composition
Optionally the combined medicine or pharmaceutical composition may be formulated into a tablet for oral delivery.

The magnitude of prophylactic or therapeutic dose of a compound of formula (I) will, of course, vary with the nature and the severity of the condition to be treated and with the particular compound of formula I and its route of administration. It will also vary according to a variety of factors including the age, weight, general health, sex, diet, time of administration, rate of excretion, drug combination and response of the individual patient. In general, the daily dose is from about 0.001 mg to about 1000 mg (e.g. 0.001 mg to about 100 mg) per kg body weight of a mammal, preferably 0.01 mg to about 10 mg per kg. On the other hand, it may be necessary to use dosages outside these limits in some cases. In
any event, the medical practitioner, or other skilled person, will be able to determine routinely the actual dosage, which will be most suitable for an individual patient.

The amount of active ingredient that may be combined with the carrier materials to produce a single dosage form will vary depending upon the host treated and the particular mode of administration. For example, a formulation intended for the oral administration of humans may contain from 0.05 mg to 5 g of active agent compounded with an appropriate and convenient amount of carrier material which may vary from about 5 to about 99.95 percent of the total composition. Dosage unit forms will generally contain between from about 0.1 mg to about 0.4 g of an active ingredient, typically 0.5 mg, 1 mg, 2 mg, 5 mg, 10 mg, 25 mg, 50 mg, 100 mg, 200 mg, or 400 mg.

Preferred doses are of compound of formula (I) are a dose of greater than 40mg daily, more preferably at least 45 mg daily.

The total daily dose may be delivered in one or more separate doses over the course of the day, alone or in combination with other therapies.

Compounds of formula (I) may be administered by any suitable means, for example orally, by inhalation spray, topically, parenterally or rectally in dosage unit formulations containing conventional non-toxic pharmaceutically acceptable carriers, adjuvants and vehicles. The term "parenteral" as used herein includes subcutaneous injections, intravenous, intramuscular, intrastemal injection or infusion techniques.

The pharmaceutical compositions containing the active ingredient may be in a form suitable for oral use, for example, as tablets, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavouring agents,
colouring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example, magnesium stearate, stearic acid or talc.

The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated to form osmotic therapeutic tablets for controlled release.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredients is mixed with water-miscible solvents such as propylene glycol, PEGs and ethanol, or an oil medium, for example peanut oil, liquid paraffin, or olive oil.

For example, a solid oral composition such as a tablet or capsule may contain from 1 to 99 % (w/w) active ingredient; from 0 to 99% (w/w) diluent or filler; from 0 to 20% (w/w) of a disintegrant; from 0 to 5% (w/w) of a lubricant; from 0 to 5% (w/w) of a flow aid; from 0 to 50% (w/w) of a granulating agent or binder; from 0 to 5% (w/w) of an antioxidant; and from 0 to 5% (w/w) of a pigment. A controlled release tablet may in addition contain from 0 to 90 % (w/w) of a release-controlling polymer.

Aqueous suspensions contain the active material in admixture with excipients suitable for the manufacture of aqueous suspensions. Such excipients are suspending agents, for example sodium carboxymethylcellulose, methylcellulose, hydroxypropyl methylcellulose, sodium alginate, polyvinylpyrrolidone, gum tragacanth and gum acacia; dispersing or wetting agents may be a naturally-occurring phosphatide, for example lecithin. The aqueous suspensions may also contain one or more preservatives, one or more colouring agents, one or more flavouring agents, and one or more sweetening agents.

A parenteral formulation (such as a solution or suspension for injection or a solution for infusion) may contain from 1 to 50 % (w/w) active ingredient; and from 50% (w/w) to 99% (w/w) of a liquid or semisolid carrier or vehicle (e.g. a solvent such as water); and 0-20% (w/w) of one or more other excipients such as buffering agents, antioxidants, suspension stabilisers, tonicity adjusting agents and preservatives.

Oily suspensions may be formulated by suspending the active ingredient in a vegetable oil, for example arachis oil, olive oil, sesame oil or coconut oil, or in mineral oil such as liquid paraffin. The oily suspensions may contain a thickening agent, for example beeswax, hard paraffin or cetyl alcohol. Sweetening agents and flavouring agents may be added to provide a palatable oral preparation. These compositions may be preserved by the addition of an anti-oxidant such as ascorbic acid, vitamin E or some such equivalent agent.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavouring and colouring agents, may also be present.

The pharmaceutical compositions of the invention may also be in the form of an oil-in-water emulsion. The oily phase may be a vegetable oil, for example olive oil or arachis oil, or a mineral oil, for example liquid paraffin or mixtures of these. Suitable emulsifying agents may be naturally-occurring phosphatides, for example soy bean, lecithin, and esters or partial esters derived from fatty acids and hexitol anhydrides, for example sorbitan monooleate, and condensation products of the said partial esters with ethylene oxide, for example
polyoxyethylene sorbitan monooleate. The emulsions may also contain sweetening and flavouring agents.

Syrups and elixirs may be formulated with sweetening agents, for example glycerol, propylene glycol, sorbitol or sucrose. Such formulations may also contain a preservative, and flavouring and colouring agents. The pharmaceutical compositions may be in the form of a sterile injectable aqueous or oleagenous suspension. This suspension may be formulated according to the known art using suitable dispersing or wetting agents and suspending agents. The sterile injectable preparation may also be a sterile injectable solution or suspension in a non-toxic parenterally-acceptable diluent or solvent, for example as a solution in 1,3-butane diol. Among the acceptable vehicles and solvents that may be employed are water, Ringer's solution and isotonic sodium chloride solution. Cosolvents such as ethanol, propylene glycol or polyethylene glycols may also be used. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Compounds of formula (I) may also be administered in the form of suppositories for rectal administration of the drug. These compositions can be prepared by mixing the drug with a suitable non-irritating excipient which is solid at ambient temperatures but liquid at the rectal temperature and will therefore melt in the rectum to release the drug. Such materials are cocoa butter and polyethylene glycols.

For topical use, creams, ointments, gels, solutions or suspensions, etc., containing the compound of formula (I) are employed. (For purposes of this application, topical application shall include mouth washes and gargles.) Topical formulations may generally be comprised of a pharmaceutical carrier, cosolvent, emulsifier, penetration enhancer, preservative system, and emollient.

### List of Figures

The invention and disclosures will now be described, by way of example only, with reference to the accompanying figures, in which:
Figure 1 is an example of a synthetic pathway for a compound VIII (also called NaQuinate herein);
Figure 2A is an example of a synthetic pathway for a compound V used in the pathway shown in Figure 1;
Figure 2B is an example of a synthetic pathway for a compound V used in the pathway shown in Figure 1;

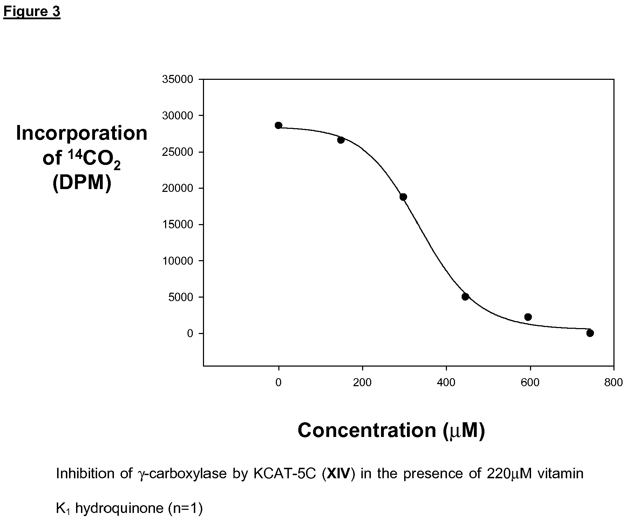

Figure 3 shows the Inhibition of g-carboxylase by KCAT-5C (XIV) in the presence of 220mM vitamin K₁ hydroquinone (n=1);

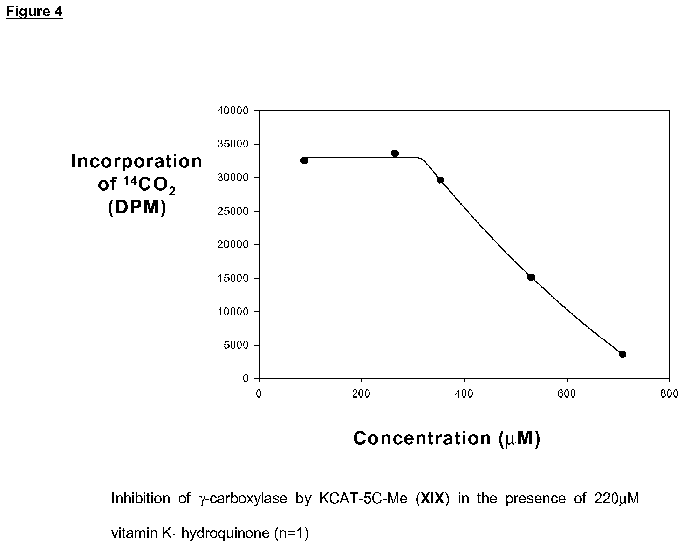

Figure 4 shows the inhibition of g-carboxylase by KCAT-5C-Me (XIX) in the presence of 220mM vitamin K₁ hydroquinone (n=1);

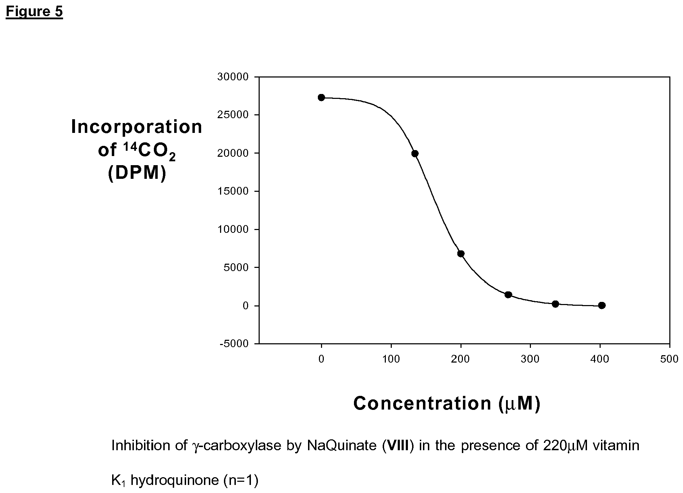

Figure 5 shows the inhibition of g-carboxylase by NaQuinate (VIII) in the presence of 220mM vitamin K₁ hydroquinone (n=1);

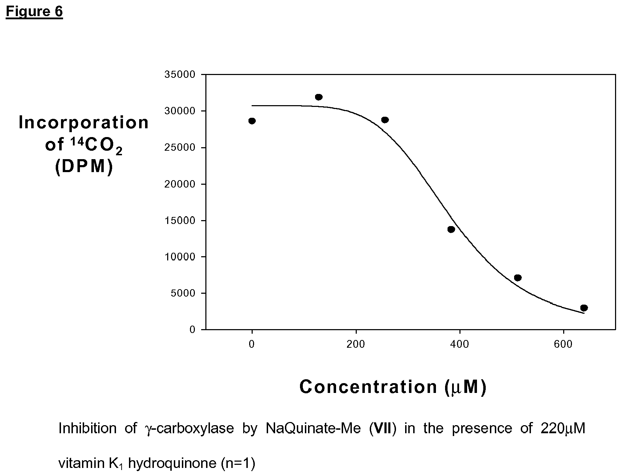

Figure 6 shows the inhibition of g-carboxylase by NaQuinate-Me (VII) in the presence of 220mM vitamin K₁ hydroquinone (n=1);

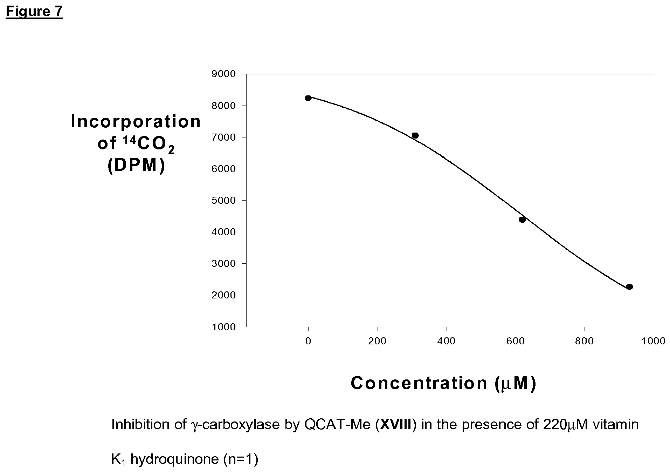

Figure 7 shows the inhibition of g-carboxylase by QCAT-Me (XVIII) in the presence of 220mM vitamin K₁ hydroquinone (n=1);
Figure 8 shows the inhibition of g-carboxylase by DMK (XVI) in the presence of 220mM vitamin K₁ hydroquinone (n=1); and
Figure 9 shows the inhibition of g-carboxylase by Vitamin K₃ (III) in the presence of 220mM vitamin K₁ hydroquinone (n=1).

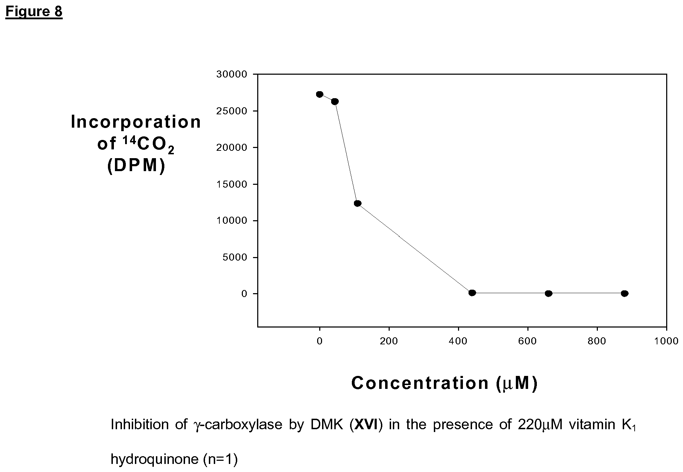

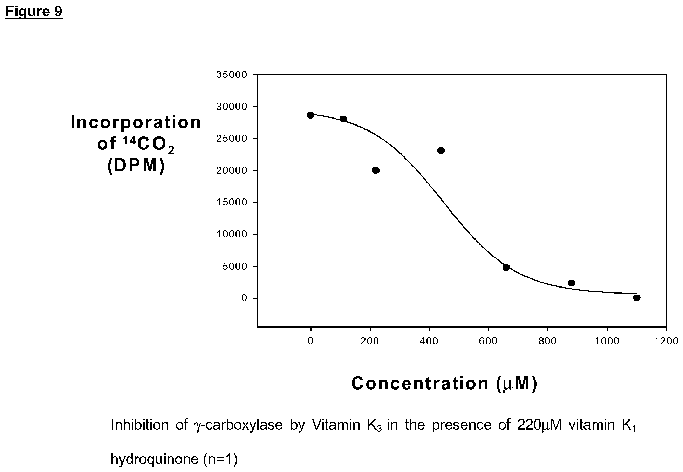

A compound according to the present invention may be synthesised by any suitable method. A suitable method is disclosed below with reference to Ruttimann et al "Chimica" (1986) 40 (9) 290-306, and Gerorkzan et al "Chem. Hetrocyclic Compd" (Engl. Trans.) (1989) 2, 269 and Figure 8 and 9. In addition, compounds of the invention may be made by analogy to the methods disclosed in GB 2,314,773. As will be
appreciated, the compounds of the current invention may be prepared by analogy to the methods disclosed in the above-mentioned references or may be bought commercially (where indicated).

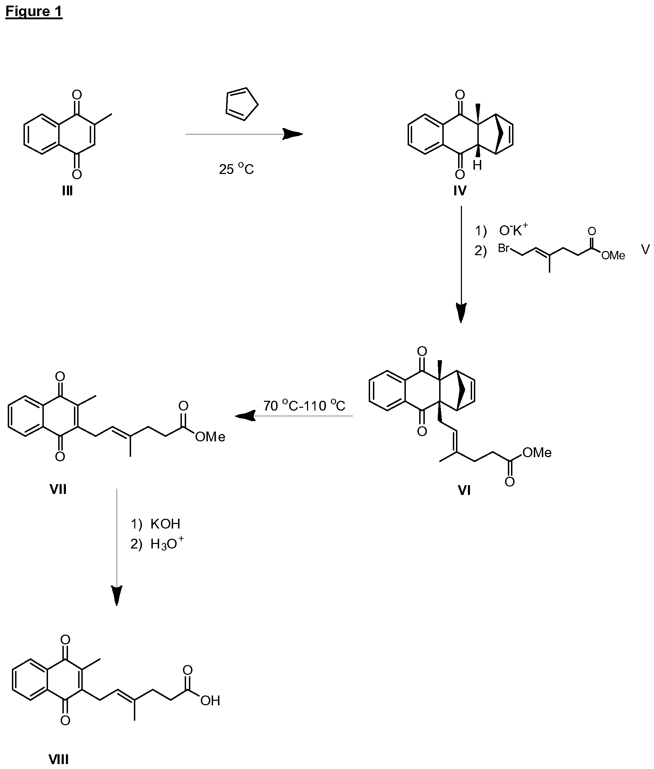

As shown in Figure 1 herein, the starting material menadione (Aldrich Chemical Company, III) was employed and reacted with cyclopentadiene at 25°C to generate the fused derivative thereof (IV). Treatment with base, O⁻K⁺ (e.g. potassium tert-butoxide) and subsequent treatment with methyl 4-methyl-6-bromo-hex-4-eneoate (V), introduced the 3-substituent (VI). The intermediate was further reacted by application of heat in the range of 70°C to 110°C causing the elimination of cyclopentadiene with the resultant isolation of the product methyl ester NaQuinate (VII). The characterisation of this compound is given by Ruttimann et al as hereinbefore referred.

The compound (VII) is converted to the corresponding carboxylic acid by means of base hydrolysis, for example using KOH, and subsequent acid treatment for example using H₃O⁺ (e.g. aqueous hydrochloric acid), or equivalent, in known manner, thereby generating NaQuinate (VIII). The characterisation of this compound is given by Ruttimann et al as hereinbefore referred.

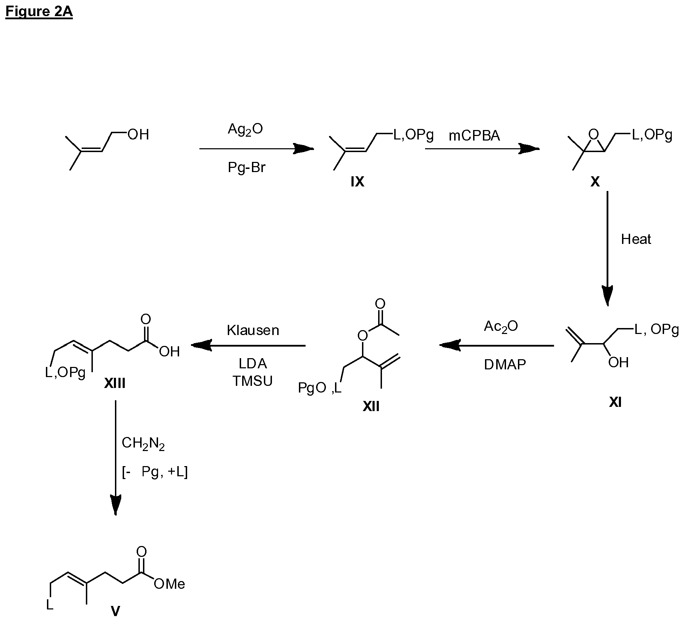

The preparation of intermediate (V) used above is carried out as shown in Figure 2A. Prenyl bromide (Aldrich Chemical Company) (IX wherein L = Br) was converted to the epoxide using mCPBA, which was subsequently heated to derive the intermediate (X) wherein L = Br. Treatment with Ac₂O-DMAP generated the ester (XII) wherein L = Br, which was then subject to Claisen (e.g. Ireland-Claisen) rearrangement using standard reagents such as LDA and TMSCI (referred to in figure 2A as TMSU). The rearranged product thereof (XIII) wherein L = Br was converted to the methyl ester (V) by reaction with CH₂N₂ for use in the preparation of NaQuinate as hereinbefore defined. The characterisation thereof is provided by Gerorkzan et al as hereinbefore referred.

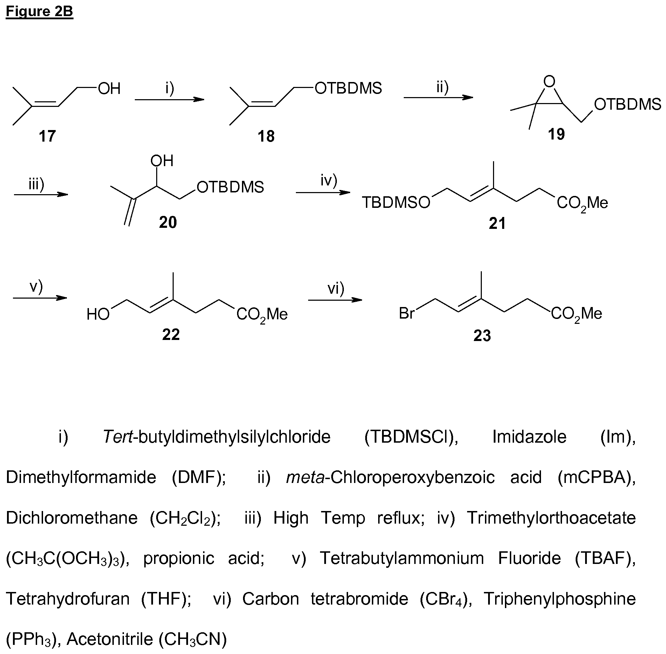

Alternatively, a more particular preparation of intermediate (V) used above is carried out as shown in Figure 2B. Prenyl alcohol (17) was protected with *tert*-butyldimethylsilylchloride (TBDMSCI) to form TBDMS ether 18. Reaction of 18 with *meta*-chloroperoxybenzoic acid formed epoxide 19, which underwent subsequent rearrangement to form alcohol 20 under high temperature reflux. Reaction of 20 with trimethylorthoacetate in the presence of propionic acid produced ester 21, which was deprotected to provide free alcohol 22 (using tetrabutylammonium fluoride). Subsequent functional group interconversion of 22 to the bromide 23 (also referred to herein as compound (V)) was achieved using carbon tetrabromide and triphenylphosphine.

Equivalent methods may be used to make other compounds within the scope of the present invention.

Determining Biological Activity: The various compounds of formula I can be tested using any of the following assays to determine their activity as anticoagulants: activated partial thromboplastin time (aPTT) test, prothrombin time (PT) test, and derivations of PT prothrombin ratio (PR) and international nationalized ratio (INR), fibrinogen testing (often by the Clauss method), platelet count, platelet function testing (often by PFA-100), TCT, and bleeding time.

The prothrombin time is most commonly measured using blood plasma. Blood is drawn into a test tube containing liquid citrate, which acts as an anticoagulant by binding the calcium in a sample. The blood is mixed, then centrifuged to separate blood cells from plasma. The plasma is analyzed on an automated instrument at 37°C, which takes a sample of the plasma. An excess of calcium is added (thereby reversing the effects of citrate), which enables the blood to clot again. Tissue factor (also known as factor III) is added, and the time the sample takes to clot is measured optically. The prothrombin time is the time it takes plasma to clot after addition of tissue factor (obtained from animals). This measures the quality of the extrinsic pathway (as well as the common pathway) of coagulation.

The INR was devised to standardize the results. The INR is the ratio of a patient's prothrombin time to a normal (control) sample, raised to the power of the ISI value for the analytical system used. Each manufacturer assigns an ISI value (International Sensitivity Index) for any tissue factor they manufacture. The ISI value indicates how a particular batch of tissue factor compares to an internationally standardized sample. The normal range for a healthy person is 0.9-1.3, and for people on warfarin therapy, 2.0-3.0, although the target INR may be higher in particular situations, such as for those with a mechanical heart valve.

For the avoidance of doubt the terms 'comprising', 'comprise' and 'comprises' herein is intended by the inventors to be optionally substitutable with the terms 'consisting of', 'consist of', and 'consists of', respectively, in every instance. The term "about" (or "around") where used in all numerical values allows for a 5% variation, i.e. a value of about 1.25% would mean from between 1.19%-1.31%.

It will be understood that particular embodiments described herein are shown by way of illustration and not as limitations of the invention. The principal features of this invention can be employed in various embodiments without departing from the scope of the invention. Those skilled in the art will recognize, or be able to ascertain using no more than routine study, numerous equivalents to the specific procedures described herein. Such equivalents are considered to be within the scope of this invention and are covered by the claims. All publications and patent applications mentioned in the specification are indicative of the level of skill of those skilled in the art to which this invention pertains.

The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the measurement, the method being employed to determine the value, or the variation that exists among the study subjects.

The term "or combinations thereof" as used herein refers to all permutations and combinations of the listed items preceding the term.

All of the compositions and/or methods disclosed and claimed herein can be made and executed without undue experimentation in light of the present disclosure. While the compositions of this invention have been described in terms of preferred embodiments, it will be apparent to those of skill in the art that variations may be applied to the compositions described herein.

Any individual aspect of the invention may be combined with any other aspect, except where apparent from the context.

The invention will be further described by reference to the following, non-limiting, examples:

### Examples

### Example 1 Observed anticoagulant activity

Mice were treated with one compound of the present invention (4E)-6-(1,4-dihydro-2-methyl-1,4-dioxonaphthalen-3-yl)-4-methylhex-4-enoic acid (**VIII**) [also called NaQuinate herein] in the form of a single injection (15µg/mouse/day) administered in 10% ethanol in saline injections intraperitoneally. Control mice received the carrier but not the NaQuinate active component.

NaQuinate treated mice were observed to bleed out much more profusely than mice that were not treated with NaQuinate, indicating that NaQuinate exerts an anticoagulant effect.

### Example 2 NaQuinate inhibits the vitamin K-dependent enzyme gamma-carboxylation reaction

The activity of NaQuinate and related compounds was examined in the vitamin K cycle.

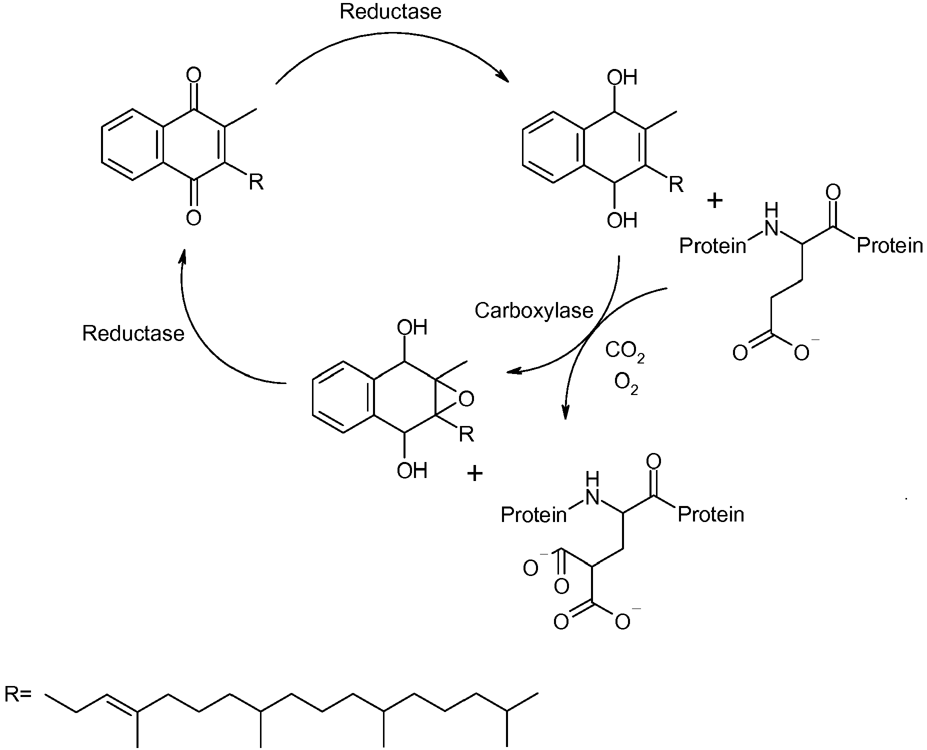

The Vitamin K cycle for Vitamin K₁

### 1. Carboxylase Assay

The method of Houben *et al* (1997) was used [Houben, R. J. et al (1997). "Assay of Vitamin K-Dependent Carboxylase Activity in Hepatic and Extrahepatic Tissues." Methods in Enzymology 282: 358-368].

All the experimental tubes were prepared by the addition of 25ml of bovine microsome preparation, 5ml of 10% 3-[(3-cholamidopropyl)dimethylammonio]-2-hydroxy-1-propanesulphonic acid (CHAPS), 25ml of saturated ammonium sulphate solution and 5ml of 0.1M dithiothreitol (DTT) (Sigma), 25ml of saturated ammonium sulphate (Sigma) solution and 5ml of 500mM decarboxylated osteocalcin (d-Oc). As the compounds were dissolved in 10ml of Dimethyl sulphoxide (DMSO), 10ml of DMSO was added to the positive and negative control tubes. Buffer D (500mM NaCl and 25mM Tris-HCI (pH 7.5)) was added to each tube to give a uniform volume of reaction mixture (125ml). The compounds (KCAT-5C (XIV), KCAT-5C-Me (XIX), NaQuinate (VIII), NaQuinate-Me (VII), QCAT-Me (XVIII), DMK (XVI) and vitamin K3 (III)) or vitamin K1 (at a concentration of 889mM) (Konakion®,mixed micelles from Hoffmann-La Roche) were added to the respective tubes apart from the negative control. The reaction was initiated immediately by the addition of 5ml of NaH¹⁴CO₃ (New England Nuclear) solution (to give a final radioactivity of 5mCi per tube) to each tube. The tubes were mixed with a vortex and placed in a water bath (20°C) for 30 minutes. 100ml of each mixture was pipetted into a vial containing 800ml of 5% (w/v) trichloroacetic acid (TCA) in order to precipitate the protein and stop the reaction. Unincorporated ¹⁴CO₂ was removed by boiling for 3 minutes. After cooling, 5ml of Optifluor was added and the level of ¹⁴CO₂ incorporation was measured on a Wallac 1414 winspectral liquid scintillation counter.

### Inhibition of Recombinant Human Gamma-Carboxylase

### Preparation of Compounds

The compounds to be tested were reduced to their hydroquinone forms by the addition of 0.2M DTT to a solution of the compounds in DMSO (giving a final v/v ratio of 1:3 DTT: DMSO) and overnight incubation in a water bath (37°C).

### Carboxylase Assay

In this assay a different microsomal preparation was used than the one described above. This microsomal preparation consists of microsomes prepared from *Trichoplusia ni* High Five cells that had had the cDNA for the human g-carboxylase incorporated into their DNA. These microsomes were taken from stores that had been prepared as described by Houben, R. J., D. Jin, et al. (1999). "Osteocalcin binds tightly to the gamma-glutamylcarboxylase at a site distinct from that of the other known vitamin K-dependent proteins." Biochem J 341 (Pt 2): 265-9.

Standard reaction mixtures contained 5ml of microsomal preparation, 5ml of 500mM decarboxylated osteocalcin (d-OC) (except for the negative control tube), 25ml of saturated ammonium sulphate solution, 5ml of 5% PC/CHAPS. In addition to this, 1:3 (v/v) DMSO: 0.2M DTT was added to the tubes (to take into account the fact that the compounds were dissolved in 1:3 DMSO: 0.2M DTT) to give a total volume of 40ul DMSO: 0.2M DTT per tube. A solution of Buffer D was added to give all final tubes a uniform volume of 125ml. The experiment was initiated by the addition of 10ml of a 1:1 (v/v) mixture of vitamin K hydroquinone (final concentration of 220mM) and NaH¹⁴CO₃ (final radioactivity of 5mCi) to each tube, followed by a range of concentrations of the compounds.

### Results

### Activation of Bovine Liver Gamma-Carboxylase

None of the compounds tested increased the incorporation of ¹⁴CO₂ into osteocalcin to a greater extent than the negative control. It can therefore be concluded that none of the compounds had any activity as a cofactor for g-carboxylase.

### Inhibition of Recombinant Human Gamma-Carboxylase

All the compounds tested were found to inhibit the g-carboxylase enzyme in the presence of reduced vitamin K1. Concentration-response curves are shown below. Results are expressed as incorporation of ¹⁴CO₂ against concentration of the compound under test.

The results are summarised as follows:

Inhibitory activity of test compounds ranked according to inhibitory activity

| Compound | IC₅₀ against 220 mM vitamin K1 (mM) |
|---|---|
| DMK (XVI) | 82.5 |
| NaQuinate (VIII) | 152 |
| KCAT-5C (XIV) | 340 |
| NaQuinate-Me (VII) | 358 |
| Vitamin K3 (III) | 460 |
| KCAT-5C-Me (XIX) | 514 |
| QCAT-Me (XVIII) | 534 |

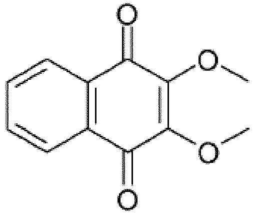

### 2,3-dimethoxy-1,4-naphthoquinone (DMK, XVI)

Vitamin K3 = menadione (III)

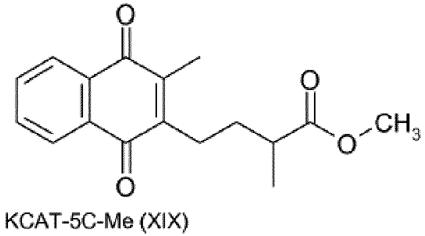

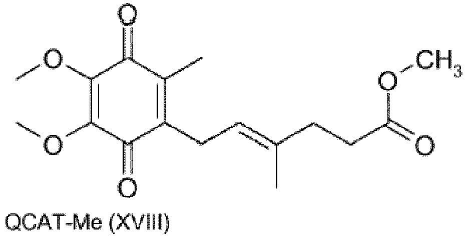

Without wishing to be bound by theory, it is believed that the anticoagulant effect observed in Example 1 is due to the inhibition (e.g. inhibition by NaQuniate) of the carboxylase enzyme in the vitamin K cycle enzyme complex.

## Claims

1. A compound of formula (I) 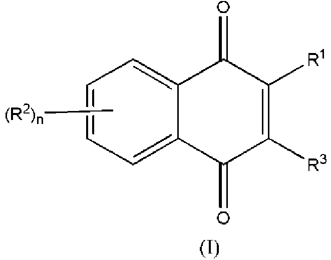 wherein:
R¹ represents hydrogen, halogen, cyano, trifluoromethyl, nitro, -OR^{a}, SR^{a}, SOR^{a}, -SO₂R^{a}, -SO₂NR^{a}R^{b}, -NR^{a}R^{b}, -NR^{a}COR^{b}, -NR^{a}CO₂R^{b}, -COR^{a}, -CO₂R^{a}, -CONR^{a}R^{b}, or a hydrocarbon group comprising a straight chain, branched or cyclic group each containing up to 18 carbon atoms, or a heterocyclic group containing up to 18 carbon atoms and at least one heteroatom;
R² represents, independently at each occurrence, hydrogen, halogen, cyano, trifluoromethyl, nitro, -OR^{a}, SR^{a}, SOR^{a}, -SO₂R^{a}, -SO₂NR^{a}R^{b}, -NR^{a}R^{b}, -NR^{a}COR^{b},-NR^{a}CO₂R^{b}, -COR^{a}, -CO₂R^{a}, -CONR^{a}R^{b}, or a hydrocarbon group comprising a straight chain, branched or cyclic group each containing up to 18 carbon atoms, or a heterocyclic group containing up to 18 carbon atoms and at least one heteroatom;
R³ is -CH₂CH=C(CH₃)(CH₂)₂CO₂H;wherein R^{a} and R^{b} independently represent, at each occurrence, hydrogen, or a hydrocarbon group comprising a straight chained, branched or cyclic group each containing up to 18 carbon atoms, or a heterocyclic group containing up to 18 carbon atoms and at least one heteroatom; and
n is 0, 1 , 2, 3 or 4;
or a pharmaceutically acceptable solvate or, more particularly, salt or prodrug thereof;
for use as an anticoagulant.

2. The compound for use according to Claim 1, wherein R¹ represents an alkyl group including straight chained or branched alkyl groups containing from 1 to 9 carbon atoms.

3. The compound for use according to Claim 1 or Claim 2, wherein:
R¹ represents a hydrocarbon group comprising a straight chain, branched or cyclic group each containing up to 18 carbon atoms, optionally wherein R¹ represents methyl.

4. The compound for use according to any one of Claims 1 to 3 wherein n represents 0.

5. The compound for use according to any one of Claims 2 or 3 wherein n represents 4 and R², at each occurrence, is hydrogen.

6. The compound for use according to claim 1, wherein the compound is (4E)-6-(1,4-dihydro-2-methyl-1,4-dioxonaphthalen-3-yl)-4-methylhex-4-enoic acid (VIII).

7. A pharmaceutical composition comprising a compound of formula (I) as defined in any one of Claims 1 to 6 and a pharmaceutically acceptable carrier, diluent or excipient therefor, for use as an anticoagulant.

8. A compound of formula (I) as defined in any one of Claims 1 to 6, or a pharmaceutical composition as defined in Claim 7,
for use in modulating the coagulation effect of a coagulant; or
for use in a method for the management of the blood clotting capability of a patient the method comprising administering to a patient in need of a treatment a therapeutically effective amount of the compound or composition to an exert an anticoagulant effect then administration of a coagulant to modulate or reverse the anticoagulant effect; or
for use in a treatment regimen comprising treatment of a patient with the compound composition (I) then treatment of the same patient with vitamin K or other coagulant following surgery to allow for blood clotting to occur.

9. A kit of parts comprising either:
components:
(i) a pharmaceutical formulation including a compound of formula (I), as defined in any one of Claims 1 to 6, in admixture with a pharmaceutically-acceptable adjuvant, diluent or carrier; and
(ii) a pharmaceutical formulation including another therapeutic agent, in admixture with a pharmaceutically-acceptable adjuvant, diluent or carrier,
which components (i) and (ii) are each provided in a form that is suitable for administration in conjunction with the other;
wherein the another therapeutic agent is selected from coagulants, optionally being vitamin K (eg vitamin K1 or vitamin K2);
or comprising
a compound of any one of Claims 1 to 6 or composition of Claim 9 and a coagulant.

10. A combination of a compound of formula (I) as defined in any one of Claims 1 to 6 or the composition of Claim 7 with a coagulant, optionally wherein the coagulant is vitamin K (eg vitamin K1 or K2).

11. A coagulant for use in modulating the effect of a compound of formula (I) as defined in claim 1-6, or a pharmaceutical composition as defined in claim 7.

## Patentansprüche

1. Verbindung der Formel (I) 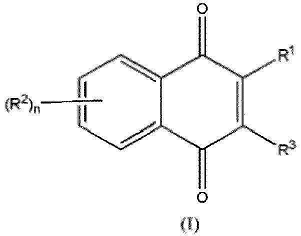 worin:
R¹ Wasserstoff, Halogen, Cyano, Trifluormethyl, Nitro, -OR^{a}, SR^{a}, SOR^{a}, -SO₂R^{a}, -SO₂NR^{a}R^{b}, -NR^{a}R^{b}, -NR^{a}COR^{b}, -NR^{a}CO₂R^{b}, -COR³, -CO₂R^{a}, -CONR^{a}R^{b} oder eine Kohlenwasserstoffgruppe darstellt, die eine geradkettige, verzweigte oder cyclische Gruppe mit jeweils bis zu 18 Kohlenstoffatomen oder eine heterocyclische Gruppe mit bis zu 18 Kohlenstoffatomen und mindestens einem Heteroatom umfasst;
R² unabhängig voneinander bei jedem Auftreten Wasserstoff, Halogen, Cyano, Trifluormethyl, Nitro, -OR^{a}, SR^{a}, SOR^{a}, -SO₂R^{a}, -SO₂NR^{a}R^{b}, -NR^{a}R^{b}, -NR^{a}COR^{b}, -NR^{a}CO₂R^{b}, -COR³, -CO₂R^{a}, -CONR^{a}R^{b} oder eine Kohlenwasserstoffgruppe darstellt, die eine geradkettige, verzweigte oder cyclische Gruppe mit jeweils bis zu 18 Kohlenstoffatomen oder eine heterocyclische Gruppe mit bis zu 18 Kohlenstoffatomen und mindestens einem Heteroatom umfasst;
R³ -CH₂CH=C(CH₃)(CH₂)₂CO₂H ist; wobei R^{a} und R^{b} unabhängig voneinander bei jedem Auftreten Wasserstoff oder eine Kohlenwasserstoffgruppe darstellen, die eine geradkettige, verzweigte oder cyclische Gruppe mit jeweils bis zu 18 Kohlenstoffatomen oder eine heterocyclische Gruppe mit bis zu 18 Kohlenstoffatomen und mindestens einem Heteroatom umfasst; und
n 0, 1, 2, 3 oder 4 ist;
oder ein pharmazeutisch verträgliches Solvat oder insbesondere Salz oder Prodrug davon; zur Verwendung als ein Antikoagulans.

2. Verbindung zur Verwendung nach Anspruch 1, wobei R¹ eine Alkylgruppe darstellt, die geradkettige oder verzweigte Alkylgruppen mit 1 bis 9 Kohlenstoffatomen beinhaltet.

3. Verbindung zur Verwendung nach Anspruch 1 oder Anspruch 2, wobei:
R¹ eine Kohlenwasserstoffgruppe darstellt, die eine geradkettige, verzweigte oder cyclische Gruppe mit jeweils bis zu 18 Kohlenstoffatomen umfasst, wobei R¹ gegebenenfalls Methyl darstellt.

4. Verbindung zur Verwendung nach einem der Ansprüche 1 bis 3, wobei n 0 darstellt.

5. Verbindung zur Verwendung nach einem der Ansprüche 2 oder 3, wobei n 4 darstellt und R² bei jedem Auftreten Wasserstoff ist.

6. Verbindung zur Verwendung nach Anspruch 1, wobei die Verbindung (4E)-6-(1,4-Dihydro-2-methyl-1,4-dioxonaphthalin-3-yl)-4-methylhex-4-ensäure (VIII) ist.

7. Pharmazeutische Zusammensetzung, umfassend eine Verbindung der Formel (I), wie in einem der Ansprüche 1 bis 6 definiert, und einen pharmazeutisch verträglichen Träger, Verdünnungsmittel oder Hilfsstoff dafür, zur Verwendung als ein Antikoagulans.

8. Verbindung der Formel (I), wie in einem der Ansprüche 1 bis 6 definiert, oder eine pharmazeutische Zusammensetzung, wie in Anspruch 7 definiert,
zur Verwendung bei der Modulation des Koagulationseffekts eines Koagulans; oder
zur Verwendung in einem Verfahren zur Handhabung der Blutgerinnungsfähigkeit eines Patienten, wobei das Verfahren die Verabreichung einer therapeutisch wirksamen Menge der Verbindung oder Zusammensetzung an einen Patienten, der einer Behandlung bedarf, um eine antikoagulierende Wirkung auszuüben, dann die Verabreichung eines Koagulans zur Modulation oder Umkehrung der antikoagulierenden Wirkung umfasst; oder
zur Verwendung in einem Behandlungsschema, umfassend die Behandlung eines Patienten mit der Verbindungszusammensetzung (I), dann die Behandlung desselben Patienten mit Vitamin K oder einem anderen Koagulans nach der Operation, um eine Blutgerinnung zu ermöglichen.

9. Kit von Teilen, umfassend entweder:
Komponenten:
(i) eine pharmazeutische Formulierung, die eine Verbindung der Formel (I), wie in einem der Ansprüche 1 bis 6 definiert, in Anmischung mit einem pharmazeutisch verträglichen Hilfsstoff, Verdünnungsmittel oder Träger beinhaltet; und
(ii) eine pharmazeutische Formulierung, die ein anderes therapeutisches Mittel in Anmischung mit einem pharmazeutisch verträglichen Hilfsstoff, Verdünnungsmittel oder Träger beinhaltet,
wobei die Komponenten (i) und (ii) jeweils in einer Form bereitgestellt werden, die für die Verabreichung in Verbindung mit den anderen geeignet ist;
wobei das andere therapeutische Mittel ausgewählt ist aus Koagulantien, die gegebenenfalls Vitamin K (z.B. Vitamin K1 oder Vitamin K2) sind;
oder umfassend
eine Verbindung nach einem der Ansprüche 1 bis 6 oder eine Zusammensetzung nach Anspruch 9 und ein Koagulans.

10. Kombination einer Verbindung der Formel (I), wie in einem der Ansprüche 1 bis 6 definiert, oder der Zusammensetzung nach Anspruch 7 mit einem Koagulans, gegebenenfalls wobei das Koagulans Vitamin K (z.B. Vitamin K1 oder K2) ist.

11. Koagulans zur Verwendung bei der Modulation der Wirkung einer Verbindung der Formel (I), wie in Anspruch 1-6 definiert, oder einer pharmazeutischen Zusammensetzung, wie in Anspruch 7 definiert.

## Revendications

1. Composé de formule (I) 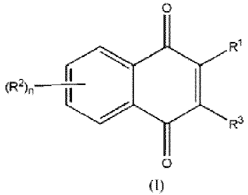 dans laquelle :
R¹ représente un hydrogène, un halogène, un cyano, un trifluorométhyle, un nitro, -OR^{a}, SR^{a}, SOR^{a}, -SO₂R^{a}, -SO₂NR^{a}R^{b}, -NR^{a}R^{b}, -NR^{a}COR^{b}, -NR^{a}CO₂R^{b}, -COR^{a}, -CO₂R^{a}, -CONR^{a}R^{b} ou un groupe hydrocarboné comprenant un groupe à chaîne linéaire, ramifiée ou cyclique contenant chacun jusqu'à 18 atomes de carbone, ou un groupe hétérocyclique contenant jusqu'à 18 atomes de carbone et au moins un hétéroatome ;
R² représente, indépendamment à chaque occurrence, un hydrogène, un halogène, un cyano, un trifluorométhyle, un nitro, -OR^{a}, SR^{a}, SOR^{a}, -SO₂R^{a}, -SO₂NR^{a}R^{b} -NR^{a}R^{b},-NR^{a}COR^{b}, -NR^{a}CO₂R^{b}, -COR^{a}, -CO₂R^{a}, -CONR^{a}R^{b} ou un groupe hydrocarboné comprenant un groupe à chaîne linéaire, ramifiée ou cyclique contenant chacun jusqu'à 18 atomes de carbone, ou un groupe hétérocyclique contenant jusqu'à 18 atomes de carbone et au moins un hétéroatome ;
R³ est -CH₂CH=C(CH₃)(CH₂)₂CO₂H ; où R^{a} et R^{b} représentent indépendamment, à chaque occurrence, un hydrogène ou un groupe hydrocarboné comprenant un groupe à chaîne linéaire, ramifiée ou cyclique, chacun contenant jusqu'à 18 atomes de carbone, ou un groupe hétérocyclique contenant jusqu'à 18 atomes de carbone et au moins un hétéroatome ; et
n vaut 0, 1, 2, 3 ou 4 ;
ou un solvate pharmaceutiquement acceptable ou, plus particulièrement, un sel ou un pro-médicament de celui-ci ;
pour une utilisation comme anticoagulant.

2. Composé pour une utilisation selon la revendication 1, dans lequel R¹ représente un groupe alkyle incluant des groupes alkyle à chaîne linéaire ou ramifiée contenant de 1 à 9 atomes de carbone.

3. Composé pour une utilisation selon la revendication 1 ou la revendication 2, dans lequel :
R¹ représente un groupe hydrocarboné comprenant un groupe à chaîne linéaire, ramifiée ou cyclique contenant chacun jusqu'à 18 atomes de carbone, optionnellement dans lequel R¹ représente un méthyle.

4. Composé pour une utilisation selon l'une quelconque des revendications 1 à 3, dans lequel n représente 0.

5. Composé pour une utilisation selon l'une quelconque des revendications 2 ou 3, dans lequel n représente 4 et R², à chaque occurrence, est un hydrogène.

6. Composé pour une utilisation selon la revendication 1, dans lequel le composé est l'acide (4E)-6-(1,4-dihydro-2-méthyl-1,4-dioxonaphtalén-3-yl)-4-méthylhex-4-énoïque (VIII).

7. Composition pharmaceutique comprenant un composé de formule (I) tel que défini dans l'une quelconque des revendications 1 à 6 et un support, diluant ou excipient pharmaceutiquement acceptable de celui-ci, pour une utilisation comme anticoagulant.

8. Composé de formule (I) tel que défini dans l'une quelconque des revendications 1 à 6, ou composition pharmaceutique telle que définie dans la revendication 7,
pour une utilisation pour moduler l'effet de coagulation d'un coagulant ; ou
pour une utilisation dans un procédé de gestion de la capacité de coagulation sanguine d'un patient, le procédé comprenant l'administration à un patient nécessitant un traitement d'une quantité thérapeutiquement efficace du composé ou de la composition afin d'exercer un effet anticoagulant, puis l'administration d'un coagulant pour moduler ou inverser l'effet anticoagulant ; ou
pour une utilisation dans un dosage de traitement comprenant le traitement d'un patient avec la composition de composé (I), puis le traitement du même patient avec de la vitamine K ou un autre coagulant après une chirurgie afin de permettre la coagulation du sang.

9. Kit de composants comprenant soit :
des composants :
(i) une formulation pharmaceutique incluant un composé de formule (I), tel que défini dans l'une quelconque des revendications 1 à 6, en mélange avec un adjuvant, un diluant ou un support pharmaceutiquement acceptable ; et
(ii) une formulation pharmaceutique incluant un autre agent thérapeutique, en mélange avec un adjuvant, un diluant ou un support pharmaceutiquement acceptable,
lesdits composants (i) et (ii) sont fournis chacun sous une forme qui convient à une administration conjointement avec l'autre ;
dans lequel l'autre agent thérapeutique est choisi parmi des coagulants, optionnellement qui sont la vitamine K (par exemple, la vitamine K1 ou la vitamine K2) ;
ou comprenant
un composé de l'une quelconque des revendications 1 à 6 ou une composition de la revendication 9 et un coagulant.

10. Combinaison d'un composé de formule (I) tel que défini dans l'une quelconque des revendications 1 à 6 ou de la composition selon la revendication 7 avec un coagulant, optionnellement dans laquelle le coagulant est la vitamine K (par exemple la vitamine K1 ou K2).

11. Coagulant pour une utilisation dans la modulation de l'effet d'un composé de formule (I) tel que défini dans les revendications 1 à 6, ou d'une composition pharmaceutique telle que définie dans la revendication 7.
